# EUROPEAN PATENT APPLICATION

(11) **EP 3 711 674 A1**
(43) Date of publication of application: **23.09.2020**
(21) Application number: 19450005.4
(22) Date of filing: 21.03.2019
(51) Int. Cl.: A61B 8/00, A61B 5/00, A61B 8/08, A61B 90/00, A61B 34/20, A61B 5/107, A61B 5/11

(54) **METHOD FOR ACQUIRING IMAGE DATA OF A BODY PART**

(71) Applicant: Medizinische Universität Wien, 1090 Wien (AT)
(72) Inventor: Kolb, Alexander, A-1140 Vienna (AT)
(74) Representative: Keschmann, Marc

(57) **Abstract**

A method for acquiring image data of a body part of a patient by means of a ultrasonography device (1) comprising the following steps:
- providing a transducer of the ultrasonography device (1), said transducer comprising a first orientation sensor (5),
- attaching a second orientation sensor (7) to the skin of the patient above the body part,
- detecting the orientation of the first orientation sensor (5) relative to the second orientation sensor (7) and verifying, whether the relative orientation corresponds to a target value,

- acquiring image data of the body part, once the relative orientation corresponds to the target value.

## Description

The invention relates to a method for acquiring image data of a body part of a patient by means of a ultrasonography device, and a device for carrying out the method.

Ultrasonography is an imaging technique for non-invasively making visible anatomical structures of the human and animal body, wherein ultrasonography is recognized as an instrument of diagnosis and intraoperative observation that is indispensable in everyday medical practice. Ultrasonography makes it possible to make observations at different depths in vivo by applying a transducer of an ultrasound device, which emits and receives ultrasound at appropriate frequencies, whereby different soft tissue layers, such as skin, adipose tissue, muscles, nerves and blood vessels as well as bone and cartilaginous structures can be seen and made available for analytic and diagnostic purposes.

Ultrasound imaging of the hip joint of infants has become a standard procedure in most industrialized countries, wherein it is routinely detected, whether the hip joint of an infant has sufficient acetabular coverage. A lack of coverage of the acetabulum is referred to in the field of hip diagnostics as dysplasia and represents a pathological condition that in the long term results in an increased wear of the hip joint due to the lack of coverage of the acetabulum and the resulting relatively poor guidance of the femoral head in the acetabulum. While dysplasia is congenital, an improvement of this condition, when diagnosed early, can be achieved in the course of the growth of the infant by applying certain orthopedic aids and therapies, in particular so-called spreader pants, with the help of which the hips of an infant are flexed and abducted and held there. By being able to significantly improve the extent of dysplasia in this relatively simple manner in the early development and early growth of an infant, hip sonography is now widely used in screening programs for hip dysplasia to be able to promptly take the necessary measures.

For the determination of the presence of dysplasia and also of the degree of dysplasia a classification was introduced by the Austrian orthopedist Prof. Reinhard Graf, in which the different morphological characteristics of a hip joint of the type I to type IV are characterized with certain sub-classifications. To determine the type of dysplasia and the sub-classifications of the different types, different angles are determined in the hip joint, which characterize the degree of acetabular coverage.

Since a transducer of an ultrasonography device reproduces the morphological structures depending on the plane, in which the transducer is directed to the body, it requires considerable experience on the part of the physician to position the transducer in a way that the relevant angles can reliably be determined. For this purpose, aids have already been used, which can be in the form of pads for positioning and stabilizing the hip of the patient and/or in the form of a fixation of the transducer, for example at right angles to the examination plane, i.e. the lying surface of the patient. The problem here, however, is that under certain circumstances, even slight deviations of the position of the patient can lead to significant deviations of the measurement results, so that there is a risk of a misdiagnosis. It is of course particularly problematic if a dysplastic hip, which could possibly be treated effectively by wearing spreader pants, is not recognized as such and the corresponding treatment subsequently fails to occur.

The invention therefore aims at improving a method for the acquisition of ultrasonographic image data so that a correct positioning and orientation of the transducer relative to the body part to be imaged, in particular the hip joint, is facilitated in order to acquire reliable imaging data and in particular to reliably assess and classify the hip joint in terms of dysplasia.

To solve this problem, the invention, according to a first aspect thereof, provides a method for acquiring image data of a body part of a patient by means of a ultrasonography device comprising the following steps:
- providing a transducer of the ultrasonography device, said transducer comprising a first orientation sensor,
- attaching a second orientation sensor to the skin of the patient above the body part,
   - detecting the orientation of the first orientation sensor relative to the second orientation sensor and verifying, whether the relative orientation corresponds to a target value,
- acquiring image data of the body part, once the relative orientation corresponds to the target value.

The inventive method is based on the idea that the relative orientation of two orientation sensors can be used by the physician to verify the correct orientation of the transducer relative to the body part of the patient that is to be inspected. In particular, it has been found that it is sufficient to attach the second orientation sensor to the skin region that covers the relevant body part instead of positioning a marker directly on or in the bone of the patient. Therefore, a non-invasive method is realized, which can be carried out within a short time and at low costs.

The orientation sensors used in the inventive method are sensors that detect the sensor's orientation in three dimensions of a three-dimensional space. Preferably, the orientation sensors may be calibrated to a common reference system, such as a Cartesian coordinate system, that is stationary to the Earth plane.

In an application of the invention, wherein the hip joint is imaged for the detection of a possible dysplasia, the second orientation sensor is attached to the skin of the patient above the pelvis, in particular above the sacrum. This mode of operation is particularly advantageous, since very few soft tissues are under the skin in the region of the sacrum, so that a reproducible plane is reliably defined, to which the ultrasound transceiver can be aligned in order to ensure proper ultrasound of the hip joint. The burden on the patient is negligible, since the attachment of the second orientation sensor is non-invasive and easy to do. When the second orientation sensor is attached to the skin of the patient in the area of the sacrum, the transducer of the ultrasound device is applied to the patient's skin in the region of the trochanter, while observing the relative orientation data of the first and second orientation sensors, and the corresponding angles can be measured by software known from the prior art in a simple manner.

When detecting the orientation of the first orientation sensor relative to the second orientation sensor it is verified, whether the relative orientation corresponds to a target value. Said target value may be set depending on the specific use case. When the method is used to detect a possible dysplasia, a preferred embodiment of the invention provides that the second orientation sensor is attached to the skin of the patient above the sacrum and the target value of the relative orientation is reached, when the first orientation sensor is aligned to the second orientation sensor at least in the cranial/caudal direction and in the ventral/dorsal direction.

Preferably, detecting the relative orientation of the first orientation sensor relative to the second orientation sensor comprises detecting a first angle between the sensors with respect to a first axis of a three-dimensional Cartesian coordinate system, a second angle between the sensors with respect to a second axis of the three-dimensional Cartesian coordinate system and optionally a third angle between the sensors with respect to a third axis of the three-dimensional Cartesian coordinate system. In this way, the transducer may be aligned in two or optionally three dimensions of the three-dimensional space. Preferably, the first and the second axis are in a sagittal plane and the third axis is oriented perpendicular to the sagittal plane of the patient. In particular, the first and the second axis are in a horizontal plane and the third axis is oriented vertically, when the patient is lying with his sagittal plane being oriented horizontally. In particular, the first angle detected with respect to the first axis is representative of a tilting position of the first orientation sensor in a cranial/caudal direction, and the second angle detected with respect to the second axis is representative of a tilting position of the first orientation sensor in a ventral/dorsal direction. The optional third angle detected with respect to the third axis is representative of a ventral/dorsal rotation of the first orientation sensor.

In particular, the target value of the relative orientation is reached, when the first angle and the second angle each are in the range of -3° to +3°, in particular 0°.

According to a preferred embodiment of the invention the sensor data from the first orientation sensor and from the second orientation sensor are transmitted to a preferably portable data processing system, wherein the step of detecting the orientation of the first orientation sensor relative to the second orientation sensor is carried out by the data processing system. Preferably, the data processing system can be integrated into mobile ultrasonography devices, such devices being widely used in paediatric practices and orthopaedic doctor's offices. The data processing system may also be provided in the form of stand-alone computers equipped with a suitable software for processing orientation data obtained from orientation sensors.

In order to simply and quickly find the correct orientation of the transducer in the course of hip sonography, the method according to the present invention may preferably be further developed such that the data processing system is designed to generate a visual, haptic and/or acoustic signal depending on the orientation of the first orientation sensor relative to the second orientation sensor. Visual signals can be generated, for example, by LEDs arranged on the transducer, and can indicate to the operator, whether the transducer has to be tilted for correct acquisition of image data, for example in the transversal plane or in the sagittal plane. Alternatively, haptic signals may be delivered in the form of vibrations. Further, acoustic signals may be generated, wherein the pitch may be modulated to indicate, if the transducer must be tilted dorsally in the transversal plane or in the opposite direction, or tilted in the cranial or caudal direction within the frontal plane.

Preferably, in a mode of operation, wherein detection the relative orientation comprises detecting a first angle between the sensors with respect to a first axis of a three-dimensional Cartesian coordinate system, a second angle between the sensors with respect to a second axis of the three-dimensional Cartesian coordinate system and optionally a third angle between the sensors with respect to a third axis of the three-dimensional Cartesian coordinate system, it may be provided that generating the visual signal comprises outputting a graphical or numeric representation of the first angle, the second angle and optionally the third angle on a graphical user interface. In this way, the operator receives precise indications into which direction to tilt or rotate the transducer in order to achieve the correct orientation. Once the operator has reached the correct orientation, he may initiate the acquisition of image data manually, for example by actuating a respective actuating element of the ultrasonography device.

Alternatively, an automatic image acquisition may be achieved by continuously detecting the orientation of the first orientation sensor relative to the second orientation sensor and comparing the relative orientation with the target value, wherein the image data of the body part is acquired automatically once the relative orientation corresponds to the target value.

Alternatively, image data is continuously acquired while the operator searches for the correct relative orientation of the transducer and the image data is stored at regular intervals so as to obtain a set of consecutive images. Once the relative orientation corresponds to the target value, the corresponding image is selected from the set for further analysis and diagnosis by the practitioner.

For precise execution of the method according to the invention, it can preferably be provided that the first and second orientation sensors are configured to detect an acceleration, a gravitational field, a magnetic field and/or an electric field. Preferably, a combination of at least two, in particular three of said physical quantities are detected, e.g. by means of a sensor that comprises a gyroscope, an accelerometer and a magnetic compass. Hall effect sensors may also be used within the scope of the invention. Such orientation sensors thus make it possible to ascertain whether the mediolateral direction of the hip of the patient is oriented vertically to the sagittal plane of the patient and whether the transducer is also aligned vertical to this sagittal plane.

If the first and/or second orientation sensor can measure acceleration values, the acquisition of image data may be suspended, when the respective sensor detects excessive movements of the patient or the transducer.

If, as it is preferred, the orientation sensors are designed to measure a magnetic field, an incorrect rotational position of the transducer relative to the patient about a vertical axis may also be detected and displayed accordingly.

According to a preferred embodiment of the present invention, an external magnetic and/or electric field is applied to a region or space, in which the first and second orientation sensors are arranged, during the acquisition of the image data. In this way a measuring plane can be defined in addition to or as an alternative to the magnetic field of the Earth, which plane is selected to allow a more precise detection of the relative orientation of the second orientation sensor. In particular, such external magnetic and/or electric field may be provided such that it is stronger than the magnetic field of the Earth so as to allow an improved detection of the sensor's orientation. Further, the external magnetic and/or electric field may be oriented so that the lines of magnetic or electric flux traverse the body part to be imaged, in particular the pelvis.

According to a second aspect of the invention, a device for acquiring image data by means of a ultrasonography device is provided, comprising a transducer having a first orientation sensor and comprising a second orientation sensor to be arranged on the patient, wherein the second orientation sensor is configured for attachment to the skin of a patient.

In order that the invention may be used with existing ultrasonography devices, retrofitting such existing devices may preferably be achieved by the first orientation sensor being fixed, preferably detachably fixed, to the transducer by means of an adapter. The adapter can in this case be made in any conceivable form, for example by injection moulding, and be easily and inexpensively adapted to various transducers commonly used on the market.

It is preferred that the second orientation sensor comprises an adhesive layer for attachment to the skin of the patient. The adhesive layer may be covered by a protective sheet prior to use. The adhesive layer will be exposed upon removal of said protective sheet and the second orientation sensor adhered to the patient's skin like a self-adhesive label.

In order to enable the most comfortable possible use of the device according to the invention, the orientation sensors are preferably wirelessly connected to a data processing system.

According to a further preferred embodiment of the invention, the data processing system is configured for receiving sensor data from the first orientation sensor and from the second orientation sensor and for detecting the orientation of the first orientation sensor relative to the second orientation sensor, wherein the data processing system is designed to generate a visual, haptic and/or acoustic signal depending on the orientation of the first orientation sensor relative to the second orientation sensor, wherein generating the visual signal preferably comprises outputting a graphical or numeric representation of the first angle, the second angle and optionally the third angle on a graphical user interface.

In particular, generating the visual signal comprises outputting a graphical or numeric representation of the first angle, the second angle and optionally the third angle on a graphical user interface.

According to a further aspect of the invention, the orientation sensors may be used in total hip arthroplasty to determine, during the implantation of the acetabular cup, its inclination and anteversion relative to the sacral plane. In this case, the first orientation sensor is attached to the setting instrument of the acetabular prosthesis instead of to the transducer. The second orientation sensor is again fixed dorsally over the pelvis, in particular over the sacrum.

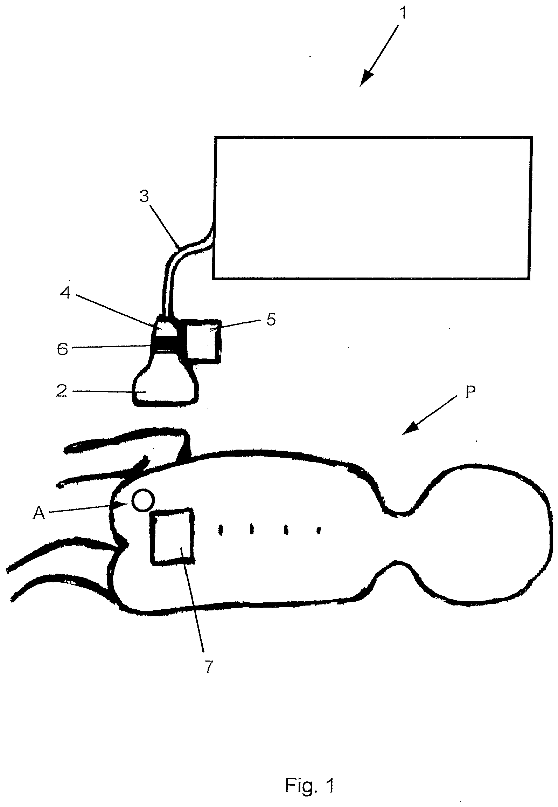

The invention will be explained in more detail with reference to an exemplary embodiment schematically shown in the drawing. Fig. 1 shows the device according to the invention in use on a patient.

In Fig. 1 the transducer of a schematically illustrated ultrasonography device 1 is denoted by 2. In the example shown in Fig. 1, the transducer 2 is connected to the ultrasonography device 1 via a cable 3 and carries a first orientation sensor 5 in a region 4. The first orientation sensor 5 is firmly connected to the transducer 2 via an adapter 6. A patient is designated by P. A second orientation sensor 7 is attached to the skin of the patient, for example, with an adhesive strip above the sacrum. The acetabulum is located approximately in the area marked with A. The relative orientation of the first orientation sensor 5 and thus of the transducer 2 to the second orientation sensor 7 and thus to the acetabulum can be determined precisely by processing the orientation data provided by the first and second orientation sensors 5 and 7. The orientation of the transducer 2 relative to the patient is adjusted by the physician until the system, based on a detection of the orientation of the first orientation sensor relative to the second orientation sensor, indicates that the ideal orientation required for the acquisition of valid images is reached. In this way, a reliable classification of the degree of dysplasia of the hip joint may be achieved.

To sum up, the invention provides a non-invasive method for guiding the practitioner to find the optimal relative orientation of a transducer of an ultrasonography device relative to a body part, wherein the absolute position of the transducer may, in addition, be found by other means, such as on the basis of the image data provided by the ultrasonography device.

## Claims

1. A method for acquiring image data of a body part of a patient by means of a ultrasonography device (1) comprising the following steps:
- providing a transducer (2) of the ultrasonography device (1), said transducer (2) comprising a first orientation sensor (5),
- attaching a second orientation sensor (7) to the skin of the patient above the body part,
- detecting the orientation of the first orientation sensor (5) relative to the second orientation sensor (7) and verifying, whether the relative orientation corresponds to a target value,
- acquiring image data of the body part, once the relative orientation corresponds to the target value.

2. The method according to claim 1, **characterized in that** the body part is the pelvis and the second orientation sensor (7) is attached to the skin of the patient above the pelvis, in particular above the sacrum.

3. The method according to claim 2, **characterized in that** the second orientation sensor (7) is attached to the skin of the patient above the sacrum and the target value of the relative orientation is preferably reached, when the first orientation sensor (5) is aligned to the second orientation sensor (7) at least in the cranial/caudal direction and in the ventral/dorsal direction..

4. The method according to claim 1, 2 or 3, **characterized in that** detecting the relative orientation of the first orientation sensor (5) relative to the second orientation sensor (7) comprises detecting a first angle between the sensors with respect to a first axis of a three-dimensional Cartesian coordinate system, a second angle between the sensors with respect to a second axis of the three-dimensional Cartesian coordinate system and optionally a third angle between the sensors with respect to a third axis of the three-dimensional Cartesian coordinate system.

5. The method according to claim 4, **characterized in that** the target value of the relative orientation is reached, when the first angle and the second angle each are in the range of -3° to +3°, in particular 0°.

6. The method according to any one of claims 1 to 5, **characterized in that** the sensor data from the first orientation sensor (5) and from the second orientation sensor (7) are transmitted to a preferably portable data processing system, wherein the step of detecting the orientation of the first orientation sensor (5) relative to the second orientation sensor (7) is carried out by the data processing system.

7. The method according to claim 6, **characterized in that** the data processing system is designed to generate a visual, haptic and/or acoustic signal depending on the orientation of the first orientation sensor (5) relative to the second orientation sensor (7).

8. The method according to claim 7, **characterized in that** generating the visual signal comprises outputting a graphical or numeric representation of the first angle, the second angle and optionally the third angle on a graphical user interface.

9. The method according to any one of claims 1 to 8, **characterized in that** the orientation of the first orientation sensor (5) relative to the second orientation sensor (7) is continuously detected and compared with the target value and that the image data of the body part is acquired automatically once the relative orientation corresponds to the target value.

10. The method according to any one of claims 1 to 9, **characterized in that** the first and second orientation sensors (5,7) are configured to detect an acceleration, a gravitational field, a magnetic field and/or an electric field.

11. The method according to any one of claims 1 to 10, **characterized in that** an external magnetic and/or electric field is applied to a region comprising the first and second orientation sensors (5,7).

12. A device for acquiring image data by means of a ultrasonography device (1), in particular for carrying out the method according to any one of claims 1 to 11, comprising a transducer (2) having a first orientation sensor (5) and comprising a second orientation sensor (7) to be arranged on the patient (P), wherein the second orientation sensor (7) is configured for attachment to the skin of a patient (P).

13. The device according to claim 12, **characterized in that** the first orientation sensor (5) is fixed, preferably detachably fixed, to the transducer (2) by means of an adapter (6).

14. The device according to claim 12 or 13, **characterized in that** the second orientation sensor (7) comprises an adhesive layer for attachment to the skin of the patient (P) .

15. The device according to claim 12, 13 or 14, **characterized in that** a data processing system is provided, which is configured for receiving sensor data from the first orientation sensor (5) and from the second orientation sensor (7) and for detecting the orientation of the first orientation sensor (5) relative to the second orientation sensor (7), wherein the data processing system is designed to generate a visual, haptic and/or acoustic signal depending on the orientation of the first orientation sensor (5) relative to the second orientation sensor (7), wherein generating the visual signal preferably comprises outputting a graphical or numeric representation of the first angle, the second angle and optionally the third angle on a graphical user interface.
